# EUROPEAN PATENT SPECIFICATION

(11) **EP 1 379 135 B1**
(45) Date of publication and mention of the grant of the patent: **04.03.2009**
(21) Application number: 02725430.9
(22) Date of filing: 29.03.2002
(51) Int. Cl.: A01N 43/04, A61K 31/70, A61K 9/127

(54) **FORMULATIONS COMPRISING ENTRAPPED ACTIVE INGREDIENTS AND USES THEREOF**
FORMULIERUNGEN, DIE EINGESCHLOSSENE WIRKSTOFFE ENTHALTEN, UND IHRE VERWENDUNG
FORMULATIONS COMPRENANT DES INGREDIENTS ACTIFS PIEGES ET UTILISATIONS DE CES FORMULATIONS

(30) Priority: 29.03.2001 US 280351 P
(43) Date of publication of application: 14.01.2004
(73) Proprietor: THE SCRIPPS RESEARCH INSTITUTE, La Jolla, CA 92037 (US); Optimer Pharmaceuticals, San Diego, CA 92121-1643 (US)
(72) Inventor: LOTZ, Martin, La Jolla, CA 92038 (US); SHIKHMAN, Alexander, R., Escondido, CA 92025 (US); HWANG, San-Bao, Sudbury, MA 01176 (US); HU, Changyong, Singapore 680250 (SG)
(74) Representative: Kaiser, Jürgen
(86) International application number: PCT/US2002/009859
(87) International publication number: WO 2002/078445

(56) References cited:
- US-A- 3 683 076
- US-A- 4 801 619
- US-A- 4 870 061
- US-A- 5 510 418
- US-A- 5 587 363
- US-A- 5 990 096

## Description

### FIELD OF THE INVENTION

This invention relates generally to the field of formulations comprising entrapped active ingredients, such as aminosugars, and uses thereof and more specifically to intra-articular injectable formulations containing N-acetylglucosamine.

### BACKGROUND

Osteoarthritis (OA) is a common joint disorder with significant societal impact (Lawrence et al., (1998) Arthritis Rheum. 41:778; Gabriel et al., (1997) J. Rheumatol. 24:719; March et al., (1997) Baillieres Clin. Rheumatol. 11:817). Classes of medications used for the treatment of OA include acetaminophens, non-steroidal anti-inflammatory drugs (NSAIDS), injectable intra-articular corticosteroids and hyaluronic acid. These drugs primarily provide pain relief, but have not yet been demonstrated to achieve true remission of the disease by slowing or otherwise halting progression of the disease (Altman et al., (1998) Osteoarthritis Cartilage 6 Suppl. A:22; Hochberg et al., (1995) Arthritis Rheum. 38:1535; Hochberg et al., (1995) Arthritis Rheum. 38:1541).

Many forms of arthritis are treated initially with NSAIDS, sometimes together with other analgesics. Where the disease is not adequately controlled with these agents, disease-modifying (remission-inducing) antirheumatic drugs, such as gold salts, D-penicillamine, antimalarial agents and cytotoxic agents, may be utilized. Ultimately, glucocorticoids may be administered, systemically or by the intra-articular route. Yet, none of these drugs is significantly effective in achieving true remission of the disease in most patients.

Bohne described the use of glucosamine (GA) to treat OA (Bohne (1969) Med. Welt 30:1668). Since then, GA has gained popularity, and now is commonly used to treat OA patients. GA salts (sulfate and chloride) are thought to have chondroprotective or disease-modifying properties (Altman et al., (1998) Osteoarthritis Cartilage 6 Suppl. A:22; Lozada et al., (1997) Bull. Rheum. Dis. 46:5; Mevorach et al., (1994) Isr. J. Med Sci. 30:928), and were originally suggested to promote the repair of damaged cartilage. Several studies have demonstrated that cartilage from patients with OA is characterized by accelerated turnover of the cartilage matrix components and by inadequate repair (Inerot et al., (1978) Biochem. J. 169:143; Dieppe et al., (1995) Acta. Orthop. Scand. (Suppl. 266) 66:1). GA has been shown to provide anti-inflammatory activity by a number of different mechanisms. For example, GA was shown to provide anti-inflammatory activity by inducing upregulation of glycosaminoglycan synthesis.

GA-induced upregulation of glycosaminoglycan synthesis represents a complex metabolic process, which is potentially mediated through several mechanisms, such as by GA directly entering the hexosamine pathway and circumventing the negative feedback control from uridine-diphosphate-N-acetyl-β-D-glucosamine (Kornfeld et al., (1964) Proc. Natl. Acad. Sci. USA 52:371) and upregulation of TGFα1 production (Kolm-Litty et al., (1998) J. Clin. Invest. 101:160). Recently, a novel mechanism of GA-mediated chondroprotection was described, which involves the inhibition of aggrecanase activity in bovine cartilage explants and rat chondrosarcoma cells (Sandy et al., (1998) Biochem. J. 335(Pt 1):59) via suppression of glycosylphosphatidylinositol-linked proteins (Sandy et al., (1999) Arch. Biochem. Biophys. 367:258).

GA may inhibit phosphorylation events in the interleukin-1β (IL-1β) signaling cascade, providing anti-inflammatory activity. One of the end-products of the hexosamine pathway, UDP-N-acetyl-β-D-glucosamine, was shown to participate in the dynamic process of protein O-glycosylation, which utilizes serine or threonine residues as anchoring sites (Haltiwanger et al., (1997) Biochem. Biophys. Res. Commun. 231:237). Potentially, O-glycosylation of serine residues can compete with phosphorylation of the same residues, resulting in impairment of intracellular signal transduction cascades (Chou et al., (1995) Proc. Natl. Acad Sci. USA 92:4417).

IL-1β is known to induce nitric oxide (NO) production in cultured human articular chondrocytes (Geng et al., (1995) J. Cell. Physiol. 163:545). IL-1β-mediated induction of certain mediators of inflammation, including NO, cyclo-oxygenase-2 enzyme (COX-2) and interleukin-6 (IL-6), is associated with translocation of transcription nuclear factor-κB (NF-κB) dimers from the cytoplasm to the nucleus, where they bind to target genes and regulate their transcription (Chu et al., (1998) Biochem. Biophys. Res. Commun. 248:871; Newton et al., (1997) FEBS Lett. 418:135; Parikh et al., (1997) J: Sur. Res. 69:139). The process of NF-κB activation depends on phosphorylation of two serines (Ser-32 and Ser-36) in the κBα inhibitory protein (IκBα) in the N-terminal regulatory domain of the κBβ inhibitory protein (IκBβ) (Karin (1999) J. Biol. Chem. 274:27339).

Anti-inflammatory mechanisms, besides GA-induced upregulation of glycosaminoglycan synthesis, may contribute to GA's anti-arthritic activities as well. GA showed anti-inflammatory activity and protected rats from paw edema induced by bradykinin, serotonin and histamine (Setnikar et al., (1991) Arzneim-Forsch./Drug Res. 41:157). GA also protected animals against serositis induced by carragenan, rat peritonitis induced by formalin, and mouse peritonitis induced by acetic acid (Setnikar et al., (1991) Arzneim-Forsch./Drug Res. 41 :157). GA did not suppress cyclooxygenase or proteolytic enzymes in the inflamed rat paw, but it did suppress superoxide generation and lysosomal enzyme activities in rat liver (Setnikar et al., (1991) Arznelm-Forch./Drug Res. 41:157).

Orally administered GA also expressed anti-inflammatory activity in kaolin- or adjuvant-induced arthritis in rats (Setnikar et al., (1991) Arzneim-Forsch./Drug Res. 41:542). However, anti-exudative and anti-inflammatory activities of GA were lower when administered orally, as compared to corresponding activities of orally administered acetylsalicylic acid or indomethacin.

A number of patents relate to the use of GA and N-acetylglucosamine (GIcNAc) for the treatment of specific arthritic conditions. U.S. Patent No. 3,683,076 (Rovati) discloses the use of GA salts for the treatment of OA and rheumatoid arthritis; U.S. Patent No. 4,870,061 (Speck) discloses the use of GIcNAc for treating degenerative joint diseases via buccal administration; U.S. Patent No. 5,840,715 and U.S. Patent No. 6,136,795 (both Florio) disclose the use of GIcNAc sulfate as a nutritional supplement in a dietary regime to provide relief from arthritis. Additionally, US 4, 801,619 (Lindblad) discloses a hyaluronic acid preparation for intra-articular administration in the treatment of steroid arthropathy and progressive cartilage degeneration caused by proteoglycan degradation.

### SUMMARY OF THE INVENTION

The present invention relates to an injectable formulation in accordance with claims 1, 9 and 12, a use of a therapeutically effective amount of an entrapped aminosugar according to claims 13 and 17, and a use of a therapeutically effective amount of N-acetylglucosamine in accordance with claim 20.
In particular, the invention relates to the anti-inflammatory and chondroprotective properties of glucosamine (GA) and N-acetylglucosamine (GIcNAc). According to this embodiment, GA and GlcNAc exhibit their anti-inflammatory and chondroprotective properties by interfering with cytokine-inducible gene expression in chondrocytes.

In one particular aspect of the present invention, GIcNAc formulations surprisingly showed unexpected analgesic effects in mammals. The analgesic properties of formulations of the invention make them useful for treating, for example, osteoarthritis (OA) or rheumatoid arthritis (RA) in a patient in need of such treatment.

In one embodiment of the method, the invention provides a method including administering to a patient a composition containing a therapeutically effective amount of GIcNAc, either alone or in combination with an existing anti-inflammatory drug or a hexoaminidase inhibitor. Suitable methods of administering formulations of the present invention include, but are not limited to, intra-articular, topical and intra-muscular methods. In one aspect where formulations are injected intra-articularly, encapsulation or entrapment of GIcNAc (as in liposomes) favorably modifies the availability and pharmacodynamic profile of intra-articularly injected GIcNAc.

### BRIEF DESCRIPTION OF THE DRAWINGS

Figure 1 shows the time-dependent release of [³H]GlcNAc entrapped in different liposomal formulations according to one embodiment of the inventions.
Figure 2 illustrates the time-dependent release of [³H] entrapped in different liposomal formulations into the synovial fluid from three rheumatoid arthritis (RA) patients.
Figure 3 illustrates the time-dependent release of [³H]GlcNAc entrapped in different liposomal formulations into the synovial fluid from three osteoarthritis (OA) patients.
Figure 4 illustrates the time-dependent retention of GlcNAc in rat knee joints after a single intra-articular administration of GlcNAc in a normal saline solution (control) as compared to a single intra-articular administration and GIcNAc entrapped in a liposomal formulation comprising DSPC/Chol.
Figure 5 illustrates the time-dependent retention of GlcNAc in rat knee joints after a single intra-articular administration of GlcNAc entrapped in a liposomal formulation comprising DSPC/Chol/DSPE.
Figure 6 illustrates a pain response (based on pain scores defined herein) for rats as a function of intra-articular administered bradykinin.
Figure 7 illustrates an analgesic effect of GlcNAc when intra-articularly (IA) administered in a liposomal formulation comprising DSPE as compared to GlcNAc in a saline solution and a saline solution.

### DETAILED DESCRIPTION OF THE INVENTION

### Abbreviations and Terms

In accordance with the present invention and as used herein, the following terms and abbreviations are defined with the following meanings, unless explicitly stated otherwise. These explanations are intended to be exemplary only. They are not intended to limit the terms as they are described or referred to throughout the specification. Rather, these explanations are meant to include any additional aspects and/or examples of the terms as described and claimed herein.

The following abbreviations are used herein:
Chol = cholesterol;
DMEM = Dulbecco's Modified Eagle Medium;
DSPC = distearoyl L-α-phosphatidylcholine;
DSPE = distearoyl L-α-phosphatidyl-ethanolamine;
DSPE-Con = distearoyl L-α-phosphatidyl-ethanolamine conjugated to polyethylene glycol
DSPG = distearoyl L-α-phosphatidyl-DL-glycerol;
DPPS = dipalmitoyl L-α-phosphatidyl-L-serine;
ERK = extracellular signal regulated kinase
GA = glucosamine
GAGs = glycosaminoglycans;
GlcNAc= N-Acetylglucosamine
HA = hyaluronic acid;
IL-1β = interleukin-1β;
IL-6 = interleukin-6;
iNOS = inducible nitric oxide synthase;
MAP = mitogen-activated protein;
MHA = Mueller-Hinton agar;
MLV = multilamellar vesicle;
NSAID = nonsteroidal anti-inflammatory drug;
OA = osteoarthritis;
PBS = phosphate-buffered saline;
PEG = polyethylene glycol;
PMSF = phenylmethylsulfonyl fluoride;
RA = rheumatoid arthritis;
SUV = small unilamellar vesicle;
TFA = Trifluoroacetic acid; and
TNFα = tumor necrosis factor.

The term "active ingredient" refers to a therapeutically effective amount of drug or formulation thereof. Preferred active ingredients of the present invention are aminosugars, such as GlcNAc and GA.

The term "alginate gel" refers to natural polysaccharide polymers comprising 1,4-linked β-D-mannuronic and α-L-guluronic acid residues in varying proportions. Alginate is capable of forming stable gels, particularly in the presence of certain divalent cations, such as calcium, barium, and strontium.

The term "aminosugar" refers to any synthetic or naturally occurring sugar wherein one or more carbon atoms are substituted with an amino group (-NH₂) in place of a hydroxyl group (-OH). Such substitution may occur without regard to orientation or configuration of any asymmetric carbons present in the sugar. Unless stated otherwise, the term "aminosugar" refers to either anomer (α or β) of a cyclic aminosugar. Aminosugars may be N-acylated, where one hydrogen atom of a pendant amino group is replaced by an acyl moiety (-COR where R = lower alkyl). According to one preferred embodiment of the invention, R = methyl (-CH₃).

The term "arthritis" refers to any particular disease characterized by joint inflammation, although the etiology of the inflammation may differ in various conditions. Relatively common arthritic diseases include rheumatoid arthritis, juvenile arthritis, ankylosing spondylitis, psoriatic arthritis and osteoarthritis. These are also referred to as "degenerative joint diseases".

The terms "articular cartilage" or "cartilage" refer to a substance that covers ends of bones and forms the joint surfaces. Cartilage can withstand compressive forces and creates a low friction surface for a joint to glide on. Articular cartilage comprises cells called chondrocytes and a matrix comprising proteins and aminosugars.

The term "cartilage degradation" refers to degradation in the tissues comprising cartilage. Such degradation is characteristic of arthritis.

The term "chitin" refers to (poly)GlcNAc linked in a β-1,4 fashion. Chitin is found throughout nature, for example in the exoskeletons of insects and crustacea.

The term "chitosan" refers to deacylated chitin, or (poly)N-glucosamine linked in a β-1,4 fashion.

The term "chondrocyte" refers to cells found within articular cartilage. Chondrocytes produce collagen, a gelatinous protein, and proteoglycans, which are glucosamine glycans linked to proteins (also called mucopolysaccharides).

The term "conjugate" refers to the combination of two or more distinct molecules that are chemically bonded. An example of a conjugate in the present invention is "DSPE-Con," wherein DSPE corresponds to distearoyl phosphatidylethanolamine conjugated to polyethylene glycol, in this case via an ester bond. Other bonds characteristic of conjugates according to the invention include, but are not limited to, amides, acetals, thioacetals, esters, and thioesters, or any such bond chiefly formed by treating a reactive carbonyl component with a nucleophile.

The terms "COX-1" and "COX-2" refer to two structurally related but functionally distinct cyclo-oxygenase enzymes. COX-1 has a homeostatic function and its inhibition is undesirable. COX-2 is an inducible enzyme whose presence increases in response to inflammation.

The term "encapsulation efficiency" refers to the amount of a compound or active ingredient encompassed, incorporated, loaded, associated, bound or otherwise entrapped within liposomes, microspheres, nanoparticles, or the like. In general, "yield" is expressed as a percent encapsulation of the active ingredient.

The term "entrapped" or "encapsulated" refers to any method of formulating an active ingredient, which confines, sequesters, or otherwise inhibits the free dissolution of the active ingredient in a solution or solid phase. Preferred examples of entrapping or encapsulating active ingredients include, but are not limited to, formulation in liposomes, microspheres, or nanoparticles, for example, solid-lipid nanoparticles.

The term "glycosaminoglycan" refers to long heteropolysaccharide molecules containing repeating disaccharide units. The disaccharide units may comprise two modified aminosugars: N-acetylgalactosamine or GlcNAc and a uronic acid such as glucuronate or iduronate. Among other functions, GAGs serve as a lubricating fluid in the joints. Specific GAGs of physiological significance are hyaluronic acid, dermatan sulfate, chondroitin sulfate, heparin, heparin sulfate, and keratan sulfate.

The term "hexosamine" refers to any aminosugar of a six-carbon polyhydroxyalcohol containing either an aldehyde or a ketone group. The term hexosamine comprises aldoses, deoxyaldoses and ketoses, without regard for orientation or configuration of the bonds of the asymmetric carbons. Preferred aminosugars are 2-, 3-, 5- or 6-deoxyketoses, preferably deoxyamino sugars such as, for example, GA, mannosamine and galactosamine. More preferably, amino sugars are N-acylated and are selected from deoxyacylamino sugars, such as, for example, GlcNAc, N-acetylmannosamine and N-acetylgalactosamine.

The term "hexosaminidase" refers to any glycosidase enzyme that partially or completely hydrolyzes chitin or chitosan into their respective monosaccharide structural units, e.g., GlcNAc and GA. Exemplary enzymes include exo-type beta-D-glucosoaminidase, beta-N-acetylhexosaminidase, chitosanase, chitinase, lysozyme, etc.

The term "hyaluronic acid" refers to a naturally occurring mucopolysaccharide comprising alternating subunits of glucuronic acid and glucosamine. Hyaluronic acid is a linear polysaccharide (long-chain biological polymer) formed by repeating disaccharide units consisting of D-glucuronic acid and N-acetyl-D-glucosamine linked by β(1-3) and β(1-4) glycosidic linkages. Hyaluronic acid is commercially available in several molecular weight ranges spanning a range from about 50,000 Daltons to about 8 x 10⁶ Daltons. Hyaluronic acid is also available as a sodium salt and is a dried, highly-purified substance. Sodium hyaluronate may be preserved with a variety of preservatives known in the art, including, but not limited to, alkyl-substituted benzoic acid esters, alcohols, conjugates, blends, and mixtures thereof.

The term "hyaluronan" refers to a polymer of repeating molecules of N-acetylglucosamine and glucuronic acid.

The term "IL-1β" refers to interleukin-1β, an immunomodulator that mediates a wide range of immune and inflammatory responses, including the activation of Band T-cells.

The term "IL-6" refers to interleukin-6, a multifunctional cytokine that is produced by a large variety of cells. IL-6 functions as a regulator of immune response, acute-phase reactions and hematopoiesis.

The term "iminocyclitol" refers to an imine analog of a sugar and is a hexosamidinase inhibitor.

The term "inflammatory arthropathy" refers to the entire collection of debilitating and painful diseases and is sometimes referred to simply as rheumatic disease. Of the various forms of inflammatory arthropathy, osteoarthritis (also referred to as osteoarthrosis) and rheumatoid arthritis are the most common. Other recognized forms of inflammatory arthropathy include ankylosing spondylitis, psoriatic arthritis, pseudogout, Reiter's Syndrome, or arthritis secondary to connective tissue diseases.

The term "injectable formulation" refers to a sterile, injectable composition prepared as a liquid solution or suspension. Solid forms suitable for solution in, or suspension in, liquid vehicles prior to injection may also be prepared. The preparation may also be emulsified or the active ingredient entrapped. An injectable formulation may also comprise a variety of preservatives known in the art, including, but not limited to, alkyl-substituted benzoic acid esters, alcohols, conjugates, blends, and mixtures thereof.

The term "intra-articular" refers to a method of delivering a drug directly to a joint. Traditional routes of drug delivery, such as for example, oral, intravenous or intramuscular administration, depend upon vascular perfusion of the synovium to carry the drug to the joint. This is inefficient because transynovial transfer of small molecules from the synovial capillaries to the joint space generally occurs by passive diffusion, which becomes less efficient with increasing size of the target molecule. Thus, the access of directing molecules, for example, GA, to the joint space is substantially restricted. Intra-articular injection of drugs circumvents those limitations.

The term "liposome" refers to vesicles that spontaneously form when, for example, phospholipids are dispersed in water or an aqueous medium, and result from the hydrophilic interaction of the lipid head groups with water and the creation of uni- and multilamellar systems (vesicles) resembling biological membranes. In a unilamellar liposome, a bilayer structure forms a hollow spherical shape with the polar sides facing an internal water compartment and external bulk water. Several acceptable methods of forming liposomes are known in the art. In general, multilamellar concentric bilayer vesicles are formed with aqueous layers separating lipid bilayers. This onion-like structure is referred to as multilamellar vesicle (MLV). Smaller unilamellar vesicles (SUVs) may be produced by sonication or extrusion of MLVs under appropriate conditions. Liposomes may be formulated with Chol for added stability and may include other materials, such as neutral lipids, and surface modifiers, such as positively or negatively charged compounds. Preferred liposomes are small unilamellar-bilayered spherical shells. Liposomes can encapsulate both lipophillic and hydrophillic drugs. When prepared by appropriate methods, they can release a drug for an extended duration. In addition, there is no toxicity associated with phospholipids. A variety of natural and synthetic phospholipids are commercially available for the preparation of liposomes. Examples known in the art and described by acronyms include, but are not limited to, DSFC, DSPE, DSPE-Con, DSPG and DPPS.

The term "microsphere" refers to a polymeric carrier used to entrap or encapsulate active ingredients of the invention. Microsphere-based formulations allow drug dosage and timing to be tailored through the choice and formulation of various active ingredient/polymer combinations. The total dose of medication and the kinetics of release are variables that can be adjusted. For example, by varying the copolymer ratio and copolymer molecular weight, drug delivery parameters can be optimized. Microsphere-based systems may also increase the life span of active ingredients. The use of microspheres comprising lactide-glycolide copolymers in formulations offers certain advantages such as biocompatability and biodegradability. Microspheres may be prepared, e.g., processed, machined, milled, ground, or extruded according to processes known in the art.

The term "percent encapsulation of drug" refers to the ratio of (1) the amount of active ingredient to be encapsulated in the final formulation of the liposome to (2) the total amount of active ingredient to be encapsulated that is used in the process of preparing the formulation prior to encapsulation, which ratio is multiplied by 100.

The terms "pharmaceutically acceptable" or "pharmacologically acceptable" refer to formulations that do not produce an adverse, allergic or other untoward reaction when administered to a mammal as appropriate, (e.g., by a physician or veterinarian).

The term "polymeric carrier"-refers to hyaluronic acid, polyethylene glycol, copolymers of polyethylene glycol and poly(lactic/glycolic acid), copolymers of poly(ethylene glycol-γ-(DL-lactic acid-co-glycolic acid), alginate gels, chitosans, or pharmaceutically acceptable salts thereof.

The terms "polyethylene glycol" and "PEG" refer to a water-soluble polymer comprising subunits HO-(CH₂CH₂O)ₙH. PEG may be end-capped with alkyl groups.

The term "solid-lipid nanoparticle" refers to a vehicle for drug delivery. Solid-lipid nanoparticles are solid solutions of drugs in a lipid matrix. Particle diameters are typically below 1 µm and the particles are typically monodispersed. Processes for preparing soiid-lipid nanoparticles may avoid the use of organic solvents and use conventional or high-pressure homogenizers and particularly suitable excipients. Due to the lipid matrix, solid-lipid nanoparticles can also stabilize chemically sensitive agents. The use of solid-lipid nanoparticles as a controlled release system is especially suitable for topical application of active ingredients.

The term "sports injury" refers to any painful or debilitating condition that is related to or caused by stress or injury incurred while engaged in sports. When arthritis develops as a result of injuries, for example sports injuries, it is generally referred to as "secondary" osteoarthritis. Cartilage degradation as a result of sports injury may be caused by a traumatic injury. In addition, other indications in which - there is cartilage damage are included herein, such as the result of other injuries or degenerative disease.

The term "sustained release" refers to the time period during which a drug is released for availability, or otherwise becomes available for physiological uptake. Periods of sustained release may be preceded by an induction period, during which little or no drug is released, or may be biphasic, comprising an initial time period during which some drug is released, and a second time period during which additional drug is released. In contrast, the term "continuous release" is used solely to describe a release profile that appears to be monophasic, having a smooth-curved time profile of release. The skilled artisan will appreciate that the release profile may actually correspond to an exponential or logarithmic time-release profile.

The term "synovial fluid" refers to a viscous, normally straw-colored substance found in small amounts in joints, bursae, and tendon sheaths. Synovial fluid comprises hyaluronic acid, a polysaccharide having a molecular weight range of 100,000-10,000,000 Daltons. Hyaluronic acid is relatively incompressible and is able to displace significant amounts of water, properties that help to make synovial fluid an excellent lubricator and shock absorber in joints.

The term "therapeutically effective amount" refers to the amount of a biologically active substance necessary to induce a desired pharmacological effect. The amount can vary greatly according to the effectiveness of a particular active substance; the age, weight, and response of the individual; as well as the nature and _ severity of the individual's symptoms. Accordingly, there is no upper or lower critical limitation with respect to the amount of the active substance. A therapeutically effective amount to be employed in the present invention can readily be determined by those skilled in the art.

The term "TNFα" refers to tumor necrosis factor, a cytokine implicated in the pathogenesis of human rheumatoid arthritis.

### Formulations and Methods

The present invention relates to GA-and GlcNAc-mediated anti-inflammatory activity. Accordingly, the following discussion details experiments designed to determine the origin of chondroprotective and anti-inflammatory properties of GlcNAc. Certain of these experiments are described in U.S. Provisional Application Serial Number 60/280,351, filed March 29, 2001, which is incorporated herein by reference in its entirety.

To investigate whether GIcNAc suppresses the enzymatic activity of inducible NO synthase (iNOS) or the expression of the corresponding protein, the effect of . GlcNAc on the expression of both iNOS protein (Western Immunoblot) and iNOS mRNA (Northern Blot) was analyzed. It was found that GlcNAc strongly inhibited the expression of both iNOS mRNA and protein. Further, it was found that both GA and GlcNAc suppressed NO production triggered by IL-1β. Both GA and GIcNAc inhibit IL-1β and TNFα induced nitric oxide (NO) production in normal human articular chondrocytes.

In order to analyze the sugar-specificity of the discovered phenomenon, the effects of glucose, glucuronic acid, N-acetylmannosamine, N-acetylgalactosamine, GIcNAc and GA on IL-1β induced NO production were compared. When used at a concentration of 10 mM, only GlcNAc and N-acetylgalactosamine demonstrated inhibitory activity. Several other monosaccharides, including glucose, glucuronic acid and N-acetylmannosamine did not inhibit IL-1β and TNFα induced nitric oxide (NO) production. Furthermore, GlcNAc polymers, including GlcNAc dimers and trimers did not express inhibitory activity against IL-1β induced NO production according to the assay and did not affect NO production.

The observed suppression of IL-1β-induced NO production is believed to be associated with inhibition of inducible NO synthase (iNOS) mRNA and protein expression. In addition, GlcNAc also suppresses the production of IL-1β-induced COX-2 and IL-6. The effect of GlcNAc on COX-2 expression in cultured human articular chondrocytes stimulated with IL-1β was studied. Results of the experiments demonstrated that GlcNAc inhibited the expression of COX-2 protein measured in the Western Immunoblot and COX-2 mRNA measured in the Northern Blot. In contrast to COX-2, GIcNAc did not affect the expression of the constitutively expressed COX-1 protein. In addition to NO and COX-2, GlcNAc inhibited IL-6 production in cultured human articular chondrocytes stimulated with IL-1β. The differences were statistically significant (p < 0.001). Therefore, GIcNAc suppresses several IL-1β-inducible products of inflammation, but does not inhibit constitutively expressed molecules.

To determine whether GlcNAc-mediated suppression of IL-1β-induced NO, COX-2 and IL-6 production depends upon suppression of the NF-κB activation, nuclear translocation of NF-κB in chondrocytes stimulated with IL-1β alone in comparison with chondrocytes stimulated with IL-1β and treated with GlcNAc was studied. Results of the experiments demonstrated that GIcNAc did not affect IL-1β-induced nuclear translocation of NF-κB. GlcNAc-mediated inhibition of the IL-1β response of human chondrocytes was not associated with the decreased activation of the MAP kinases (JNK, ERK and p38) or with activation of the transcription factor NF-κB.

Intracellular signaling in the IL-1β pathway results in activation of several protein kinases, including the MAP kinases (Geng et al., (1996) J. Clin. Invest. 98:2425:30). GlcNAc residues participate in the dynamic process of protein O-glycosylation, which utilizes serine residues as anchoring sites. Therefore, by competing for the same binding sites, O-glycosyl residues could diminish the efficacy of serine phosphorylation and, thus, interfere with signal transduction. To address this potential mechanism, the effect of.GlcNAc on MAP kinase (ERK, JNK and p38) activation in chondrocytes induced by IL-1β was analyzed. Results of these experiments demonstrated that GlcNAc does not inhibit ERK, INK and p38 MAP kinase activation.

GlcNAc did not suppress all responses in chondrocytes induced by IL-1β. For example, it did not suppress the IL-1β mediated increase in hexosaminidase secretion. Moreover, it was synergistic with IL-1β in the induction of TGFα1. Collectively, these findings suggest that GlcNAc selectively inhibits cytokine-induced gene expression and the production of certain pro-inflammatory mediators.

Results of the experiments showed that both GA and GIcNAc measurably inhibited NO production in the lower millimolar range; however, concentrations below 1 mM were not effective. This concentration range is similar to that previously described for GA-induced upregulation of TGFα production in cultured porcine mesangial cells (Kolm-Litty et al., (1998) J. Clin. Invest. 101:160). Better anti-inflammatory activity at higher concentrations of GA and GlcNAc may reflect a competition between these sugars and glucose from culture media for entering the cells via glucose transporter molecules (Rauchman et al., (1992) Biochim. Biophys. Acta. 1111 :231). Therapeutic concentrations of the aminosugars, which can be reached in humans upon oral administration of GA at the accepted dose of 1,500 mg a day, are much lower than those used in the present publication. Therefore, the *in vitro* data regarding the anti-inflammatory mechanisms of GlcNAc and GA activities cannot be directly applied to explain the therapeutic efficacy of GA in patients with OA.

When used in equimolar concentrations, GlcNAc demonstrated stronger inhibition of NO production than GA. Maximal inhibitory effect of GlcNAc was observed with a concentration of 20 mM; concentrations lower than 1 mM were insufficient in the suppression of NO production. The IC₅₀ for GlcNAc was 4.1 ± 1.3 mM, the IC₅₀ for GA was 14.9 ± 2.1 mM, (p < 0.01). Neither GA nor GlcNAc at dosages up to 20 mM affected cell viability as measured by a known MTT assay (Park et al., (1987) Cancer Res. 47:5875).

While not wanting to be bound by a particular theory, a possible mechanism for GlcNAc-mediated inhibition of the IL-1β response is a N-acetylglucoamine-mediated inhibition ofphosphorylation events in the IL-1β signaling cascade. One of the end-products of the hexosamine pathway, UDP-N-acetyl-β-D-glucosamine, was shown to participate in the dynamic process of protein O-glycosylation, which utilizes serine or threonine residues as anchoring sides (Haltiwanger et al., (1997) Biochem. Biophys. Res. Commun. 231:237). Potentially, O-glycosylation of the serine residues can compete with the phosphorylation of the same residues resulting in the impairment of intracellular signal transduction cascades (Chou et al., (1995) Proc. Natl. Acad. Sci. USA 92:4417). To address this possibility, the effect of GlcNAc on MAP kinase (ERK, JNK and p38) activation, and on nuclear translocation of NF-κB in chondrocytes stimulated by IL-1β was analyzed. The activation of these MAP kinases and of NF-κB are central events in the chondrocyte response to IL-1β and related cytokines. Results of the experiments revealed that measurable GlcNAc did not inhibit IL-1β-induced activation of MAP kinases (ERK, JNK and p38), or the nuclear translocation of NF-κB.

To summarize, the present study was the first to examine the effect of GA and GlcNAc on human chondrocyte response toward the stimulation with IL-1β, and it describes a novel mechanism of GlcNAc-mediated anti-inflammatory activity. Results of our experiments clearly indicated that GA, and to a greater degree, GlcNAc are capable of inhibiting IL-1 β-induced NO production in cultured human articular chondrocytes. The effect of sugars on NO production is specific since several other monosaccharides, including glucose, glucuronic acid and N-acetyl-mannosamine do not express this activity. Furthermore, it was demonstrated that GlcNAc polymers, including the dimer and the trimer, also do not affect NO production. The observed suppression of IL-1β induced NO production is the consequence of inhibition of iNOS protein and mRNA expression. In addition to its NO inhibitory activity, GlcNAc also suppressed the production of IL-1β-induced COX-2 and IL-6. The expression of COX-1, however, was not affected by GlcNAc.

One embodiment of the present invention provides methods for treating OA (or types of inflammatory arthropathy), sports injuries, or cartilage degradation, in a patient in need of such treatment. The method includes the step of administering to a patient a composition containing a therapeutically effective amount of GlcNAc alone or in combination with at least one other anti-inflammatory drug or a hexoaminidase inhibitor, which anti-inflammatory drugs and inhibitors are known in the art. Additional embodiments of the present invention involve formulations suitable for intra-articular, topical, or intra-muscular administration. Advantageously, the formulations can be effectively administered intra-articularly.

The preparation of a pharmacological composition that contains active ingredients dissolved or dispersed therein need not be limited based on formulation. Such compositions may be prepared as injectable solutions either as liquid solutions or suspensions. However, solid forms suitable for dissolution, or resuspension, in liquid prior to use can also be prepared. The preparation can also be emulsified.

The active ingredient can be mixed with excipients that are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances, such as wetting or emulsifying agents, pH buffering agents and the like, which enhance the effectiveness of the active ingredient.

Compositions of the present invention can include pharmaceutically acceptable salts of the components therein. Pharmaceutically acceptable salts include acid addition salts (formed with any free amino groups) that are formed with inorganic acids such as, for example, hydrochloric or phosphoric, sulfuric acids, etc.) or such organic acids as acetic, tartaric, mandelic and the like. Salts formed with the free carboxyl groups present in glucoglycans can also be derived from inorganic bases such as, for example, sodium, potassium, ammonium, calcium or ferric hydroxides, and such organic bases as isopropylamine, trimethylamine, 2-aminoethanol, histidine, procaine and the like. Particularly preferred are the salts of TFA and HCl.

Another embodiment of the present invention is an improved formulation for the active ingredient, GlcNAc. Encapsulation or entrapment of GlcNAc in liposomes or other entrapping agents modifies its pharmacodynamic profile when intra-articularly injected. For example, aqueous solutions of intra-articularly administered GlcNAc move rapidly out of the rat knee joints. In contrast, GlcNAc formulated in liposomes is retained in rat knee joints after intra-articular administration according to the invention. In another embodiment of the invention, a liposomal formulation of GlcNAc showed unexpected analgesic effects on bradykinin-induced pain in a mammal. GlcNAc itself, without liposomal formulation, showed small, but statistically insignificant, analgesic effects.

The invention will be further described with reference to the following examples; however, it is to be understood that the invention is not limited to such examples.

### EXAMPLES

### Materials

Normal cartilage was obtained from autopsy services and tissue banks in San Diego, Orange County and San Antonio. Articular cartilage was harvested from femoral condyles and tibial plateaus. All tissue samples were graded according to a modified Mankin scale (Mankin et al., (1971) J. Bone Joint Surg Am. 53:23), and only cartilage without evidence of OA was used as a source of chondrocytes. The interval between death and the time the cartilage was harvested from these knee joints in the laboratory was at least 24 hrs and ranged up to 96 hrs. Cartilage shavings were harvested by the tissue banks within 24 hrs post mortem, placed in tissue culture medium (DMEM, 10% FBS, Penicillin, Streptomycin) and shipped to the laboratory at 4°C. This tissue was processed in the laboratory within 24 hrs after harvest.

GA, GlcNAc, glucose, glucuronic acid, and N-acetylmannosamine were purchased from Sigma (St. Louis, MO). N-acetylglucoamine dimer (N,N'-diacetylchitobiose) and N-acetylglucoamine trimer (N,N',N"-triacetylchitotriose) were purchased from TRC (Toronto, Canada).

Equipment and animals used in formulation studies were commercially available: Circulating water bath (Huber), vacuum-pump (Gast manufacturing Inc, Model DOA-P104-BN), multi-purpose scintillation counter (Beckman coulter, LCS-6500), SpeedVac concentrator (Savant, Model DNA-230). Wistar rats (male, 200-250 g) used in the rat experiments were obtained from the Singapore Laboratory Animal Center.

### Methods

Chondrocytes were isolated from the cartilage by collagenase digestion and maintained in continuous monolayer cultures in DMEM containing 10% fetal bovine serum (FBS). Cell viability after chondrocyte isolation by collagenase digestion of normal cartilage was >95%. This level was maintained for at least 96 hrs post mortem. Studies on IL-1 effects as a catabolic response showed no apparent changes as a function of variations in the time between death and tissue processing when nitric oxide and IL-6 release were measured. Experiments reported were performed with primary or first passage cells.

Chondrocytes were plated at 40,000 cells/well in 96-well plates in the presence of 1 % FBS. After 48 h, the medium was changed, and the cells were stimulated with IL-1β (Sigma) at a concentration of 5 ng/ml for 24 h. NO production was detected as NO₂ accumulation in the culture supernatants by the Griess reaction as described elsewhere (Heval et al., (1994) Methods Enzymol. 233:250). IL-6 in the culture supernatants was measured by ELISA (R&D Systems, Minneapolis, MN) in accordance with the supplier's protocol.

Whole cell extracts were prepared from 3 x 106 chondrocytes stimulated as described above by lysing the cells on the plate with ice cold lysis buffer (10 mM TrisHCl pH 7.6, 158 mM NaCl, 1 mM EDTA, 0.1% SDS, 1% Triton X-100, leupeptin lllg/ml, aprotinin 1µg/ml, and 0.5 mM PMSF), which was added immediately before use. The lysates were transferred to Eppendorf tubes and centrifuged at 20,000 g for 30 min at 4 °C. The supernatants were transferred into fresh tubes, and the protein concentration was determined by Bradford assay. Similar amounts of protein were separated by 10% SDS PAGE and transferred to a nitrocellulose filter (Schleicher & Schuell, Keene, NH) by electroblotting. The filter was blocked overnight in 5% milk powder/TBS-T solution and then further incubated with one of the following antibodies: anti-iNOS (C-19, Santa Cruz Biotechnology, Inc., Santa Cruz, CA), anti-COX-2 (Cayman Chemical, Ann Arbor, MI), anti-COX-1 (H-3, Santa Cruz Biotechnology, Inc.), anti-phospho-JNK (phosphoThr 183 /Tyr 185, New England Biolabs, Inc., Beverly, MA), anti-phospho-p38MAP (phospho-Thr 80/Tyr 182, New England Biolabs, Inc.) or anti-phospho-ERK (phosphoThr 180/Tyr 182, New England Biolabs, Inc.) for 2 hrs. The membranes were washed 3 times with TBS-T and then further incubated with the appropriate horseradish peroxidase labeled secondary antibody in 5% milk powder/TBS-T and developed using ECL system (Amersham, Arlington Heights, IL).

Total RNA was isolated from 2 x 106 chondrocytes stimulated using the STAT-60 reagent (Tel-Test, Friendswood, TX). RNA for each sample was quantified photometrically, and 5 µg was separated on 1.2% agarose, 6% formaldehyde gels. After electrophoresis, the gels were photographed, and the RNA was transferred onto HybondN nylon membranes (BRL, Baithesburg, MD) by capillary blotting. Membranes were air-dried and incubated for 2 hrs at 80°C. Prehybridization was done for 2 hrs at 60°C in 5 x SCC, 1 mM EDTA, 0.2% SDS and 5 x Denhardt solution. Radio-labeled probe was added and hybridization was carried out overnight at 60°C. After hybridization, the filters were rinsed twice in 2 x SSC, 0.1% SDS, washed once in 2 x SCC, 0.1 % SDS at 60°C and once in 0.2 x SCC, 0.1 % SDS at 60°C. The membranes were covered with plastic wrap and exposed with an intensifying screen for 12 hr at -70°C. The probes used for the hybridization were prepared as described earlier (Geng et al., (1993) J. Immunol. 151:6692; Geller et al., (1993) Proc. Natl. Acad. Sci. USA 90:3491).

Nuclear protein extracts were prepared as follows: 2 x 10⁶ chondrocytes were stimulated as indicated. The cells were harvested by trypsinization, washed once with ice cold PBS and lysed in 10 mM Tris-HCl buffer pH 7.5 containing 2 mM MgCl₂, 140 mM NaCl, 0.5 mM DTT, 0.05% Triton X-100, 0.5 mM PMSF, leupeptin 1 µg/ml and aprotinin 1 µg/ml. The nuclei were spun down, resuspended in 20 mM HEPES buffer pH 7.9, containing 25% glycerol, 420 mM NaCl, 1.5 mM MgCl₂, 0.2 mM EDTA, 0.5 mM DTT, 0.5 mM PMSF, leupeptin 1 µg/ml and aprotinin 1 µg/ml, and rotated at 4°C for 30 minutes. After removal of the nuclear debris by centrifugation, the protein concentration of the lysate was determined by Bradford assay. Equal amounts of the nuclear extracts (2 µg) were incubated for 15 minutes at room temperature with poly-(dI-dC)poly-(dI-dC) (0.1 mg/ml), BSA (1 mg/ml), 1 x 1Os counts of double-stranded radiolabeled oligodeoxynucleotide containing the NF-κB consensus DNA binding site (sequence: 5'-GATCGAGGGGACTTTCCCTAGC-3') in 20 mM HEPES buffer pH 7.9 containing 10% glycerol, 420 mM NaCl, 1.5 mM MgCl₂, 0.2 mM EDTA, 0.5 mM DTT and 0.5 mM PMSF. For competition experiments, unlabeled NF-κB oligodeoxynucleotide or oligodeoxynucleotide containing the Oct-1 consensus sequence were added at 100-fold molar excess to the binding reactions 10 minutes prior to the addition of radiolabeled NFκB oligodeoxynucleotide. The binding reactions were loaded onto 6% TGE (50 mM Tris-HCl pH 7.5, 380 mM glycine, 2 mM EDTA) native polyacrylamide gel and electrophoresed for 2 hrs at 4°C. The gels were then dried and exposed for 16 to 48 hrs with intensifying screen at -80°C. Statistical analysis of the generated data was performed with the aid of StatMost 32 program for Windows (Dataxiom Software Inc., Los Angeles, CA).

The following phospholipids and phospholipid conjugates are commercially available. Commericial names are given, followed by vendor and product number, together with acronym in parentheses:
distearoyl L-α-phosphatidylcholine (Sigma P6517, DSPC), distearoyl L-α-phosphatidyl-ethanolamine (Sigma P3531, DSPE), distearoyl L-α-phosphatidylethanolamine conjugated with methoxypolyethylene glycol (Sigma P7840, DSPE-Con), distearoyl L-α-phosphatidyl-DL-glycerol (Sigma P9524, DSPG), dipalmitoyl L-α-phosphatidyl-L-serine (Sigma P1185, DPPS), cholesterol (Sigma C8667, Chol), N-acetylglucosamine (Sigma A8625, GlcNAc).

Tritiated N-glucosamine, N-acetyl-D-[1-³H]glucosamine was obtained from Amersham Pharmacia Biotech, Code TRK376, specific activity 18.6 mCi/mg, radiochemical purity 97.4%). Solvents and reagents, chloroform (Sigma, C-2432), methanol (Merck, HPLC grade), NCS-II tissue solubilizer (Amersham Pharmacia Biotech, Code NNCS502), scintillation cocktail (Amersham Pharmacia Biotech, Code BCS-NA), were used as received unless otherwise noted.

### EXAMPLE 1

### PREPARATION OF GlcNAc ENTRAPPED BY LIPOSOMES

33 µmol DSPC and 33 µmol Chol (or other compositions, see Table 1 below) were dissolved in 3 ml of chloroform:methanol (95:5, v/v) solution in a 100-ml Erlenmeyer flask. The organic solvent was evaporated under vacuum (600 mm Hg) at room temperature. After the last visible traces of solvent had been removed, vacuum was continued at least for another 30 min.

2 ml of GlcNAc in 0.85% sodium chloride solution (containing a total of 10 µCi labeled and 5.38 mg unlabeled GlcNAc with a final unlabeled to labeled molecular ratio of 10,000 tol) was added into a flask. The flask was left in a 60°C (temperatures for other compositions are shown in Table 1) water bath for 5 min and the dry lipid was suspended by vigorously vortexing at 2,500 rpm for 1 min. Liposomes were left to anneal at the same temperature for 45 min. The prepared liposomes were then pipetted into four 1.5-ml Eppendorf tubes. Liposomes were dried under vacuum in a SpeedVac concentrator at 45 °C for 4-5 hrs.

50 µl of distilled water was added to the dried liposomes in each Eppendorf tube. The Eppendorf tubes were left in a 60°C (The temperatures for other compositions were shown in Table 1.) water bath for 5 min. The liposomes were then suspended by vortexing at 2,500 rpm for 1 min. The suspension was left to stand at the same temperature for 45 min.

The non-encapsulated GlcNAc was then separated from the liposomes and GlcNAc encapsulated in liposomes by centrifuging for 15 min at 20,000 G after diluting with 1 ml 0.85% NaCl. The same process was repeated an additional two times with 1 ml 0.85% NaCl to remove the non-entrapped material. The following liposomal formulations were prepared according to this method:
(1) A binary mixture comprising DSPC and Chol in a molar ratio of 5:5;
(2) a ternary mixture comprising DSPC, Chol and DSPE in a molar ratio of 5:5:1;
(3) a ternary mixture comprising DSPC, Chol and DSPE-Con in a molar ratio 5:5:1;
(4) a ternary mixture comprising DSPC, Chol and DSPG in a molar ratio of 5:5:1; and
(5) a ternary mixture comprising DSPC, Chol and DPPS in a molar ratio of 5:5:1.

The amount of [³H]GlcNAc in each liposome formulation and corresponding supernatant was determined by liquid scintillation counting. Briefly, 10 µl liposome solution or supernatant was dissolved in 0.5 ml tissue solubilizer (Amersham NCS-II) and mixed with 15 µl glacial acetic acid to eliminate possible background chemiluminescence. The supernatant or liposomes were then mixed with 4.5 ml of scintillation cocktail (BCS-NA, Amersham). Radioactivity in the liposome pellet and supernatant was used for the calculation of the encapsulation efficacy for each liposome preparation.

Table 1 shows the encapsulation efficacy of GlcNAc in various liposomal formulations. Yield means the amount of the compound incorporated, loaded, associated, bound or otherwise attached to the liposomes or their bilayers. In general, the yield is expressed in % of the starting amount. As shown in Table 1, the efficacy of GlcNAc encapsulation into liposomes is consistent. The yield values in Table 1 are representative values of percent encapsulation (yield). Other values (higher or lower) may be obtained according to the type and amounts of lipids employed, without departing from the spirit of the invention.

**Table 1**

| LIPOSOME FORMULATIONS OF GIcNAc | | | |
|---|---|---|---|
| DSPC/Chol | Annealing and rehydration temperature | | Yield (%) |
| | 60 °C | | 20.4 |
| | | | 20.0 |
| | | | 23.3 |
| | | | 16.0 |
| | | Mean±SD | 19.9±3.0 |

| DSPC/Chol/DSPE (5:5:1) | Annealing and rehydration temperature | | Yield (%) |
|---|---|---|---|
| | 75 °C | | 23.6 |
| | | | 18.8 |
| | | | 30.6 |
| | | | 27.6 |
| | | Mean±SD | 25.2±5.1 |

| DSPC/Chol/DSPE-Con (33 µmol:33 µmol:20 mg) | Annealing and rehydration temperature | | Yield (%) |
|---|---|---|---|
| | 75 °C | | 19.2 |
| | | | 18.6 |
| | | | 23.9 |
| | | | 17.5 |
| | | Mean±SD | 19.8±2.8 |

| DSPC/Chol/DSPG (5:5:1) | Annealing and rehydration temperature | | Yield (%) |
|---|---|---|---|
| | 60 °C | | 28.4 |
| | | | 25.4 |
| | | | 21.8 |
| | | Mean ± SD | 25.2 ± 3.3 |

| DSPC/Chol/DPPS (5:5:1) | Annealing and rehydration temperature | | Yield (%). |
|---|---|---|---|
| | 75 °C | | 15.2 |
| | | | 19.4 |
| | | | 23.0 |
| | | | 33.2 |
| | | Mean ± SD | 22.7 ± 7.7 |

All the preparation procedures were performed in a sterile fashion. The sterility of prepared liposomes was checked using an agar bacterial culture plate. 5 µl of liposomes were inoculated on an agar plate and cultured at 35 °C for 3 days. Another 5 µl of liposomes were inoculated onto an MHA agar plate and cultured at room temperature for 3 days. They all showed no growth of any organisms.

### EXAMPLE 2

### IN VITRO RELEASE OF GlcNAc FROM LIPOSOMES

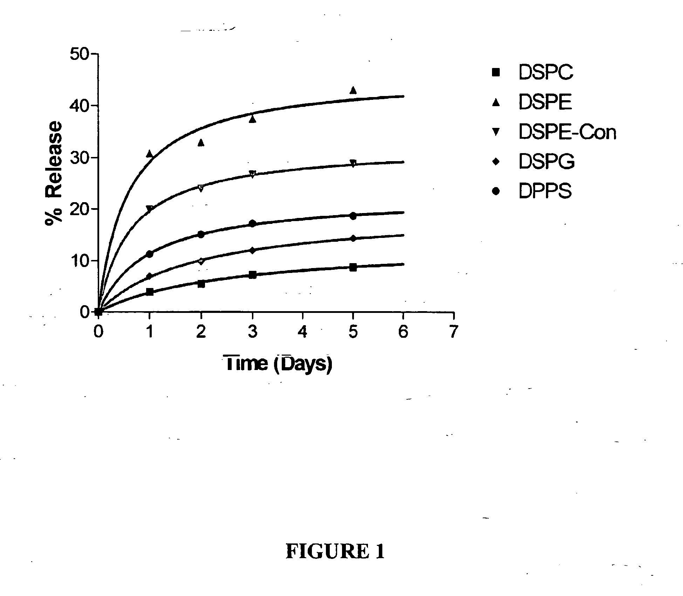

100 µl GlcNAc-encapsulated in liposomes prepared as above was suspended in 3 ml of 0.1 M PBS (pH 7.4) in a 50 ml centrifuge tube. The liposomes were incubated in a shaker incubator at 60 rpm and 37 °C. At different time points, the liposomes were separated by centrifugation at 20,000 rpm for 15 min. The released GlcNAc (³H activity) in the supernatant was measured by liquid scintillation counter. The liposome pellets were re-suspended in 3 ml of fresh PBS and incubated in the same conditions. The percent release of [³H]GlcNAc as a function of time for various types of liposomes is plotted in Figure 1.

Figure 1 shows a kinetic profile for the in *vitro* release of [³H]GlcNAc entrapped in different liposomal formulations in the presence of 0.1 M PBS, pH 7.4. The amount of [³H]GlcNAc released from liposomes was measured in the supernatant and calculated as the percentage of the initially entrapped compound released.

Figure 1 reveals that the rate of release of [³H]GlcNAc varies according to the type of liposome employed. In these experiments, the liposomal formulation employing DSPE was characterized by a rapid initial rate of release followed by a slower rate of release at later times. A similar formulation incorporating DSPE conjugated to polyethylene glycol shows markedly different behavior, showing significantly slower release of drug at the same point in time as the formulation lacking the PEG conjugate. Liposomes comprising DSPC showed a slow, nearly steady rate of release. The other formulations appeared intermediate between that of DSPE and DSPC.

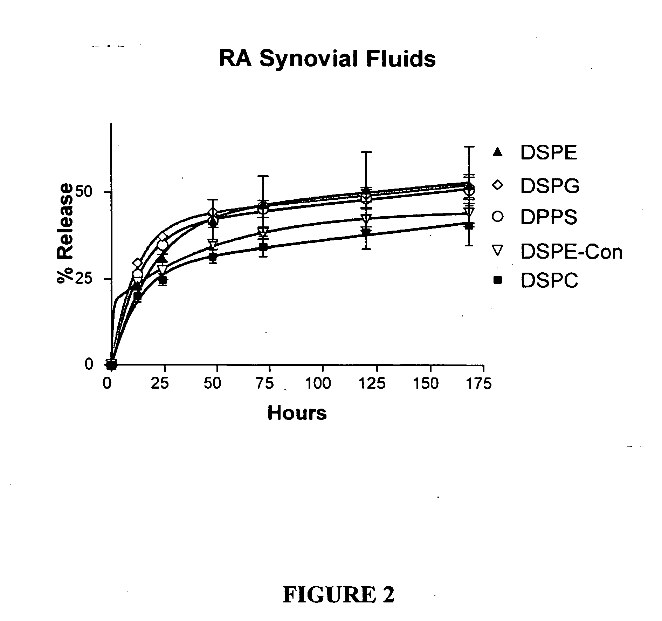

An in vitro release study was also performed in human synovial fluids from inflammatory arthritis and OA patients. 10 µl liposomes, prepared as described herein, were mixed with 90 µl human synovial fluids. They were incubated in a shaker incubator at 60 rpm and 37 °C. At different time points, the liposomes were separated by adding 300 µl normal saline and centrifuged at 20,000 G for 15 min. The supernatant was added into 4 ml liquid scintillation cocktail and the released GlcNAc (³H activity) was measured by liquid scintillation counter. The liposome pellets were re-suspended in 90 µl human synovial fluids from the same patients and incubated under the same conditions as described above. The percent release of [³H]GlcNAc as a function of time for various types of liposomes in the presence of synovial fluid is plotted in Figure 2.

Figure 2 shows a kinetic profile for *in vitro* release of [³H]GlcNAc entrapped in different liposomal formulations into the synovial fluid from 3 rheumatoid arthritis (RA) patients. The amount of [³H]GlcNAc released from liposomes was measured in the supernatant and calculated as the percentage of the initially-entrapped compound released. In Fig. 2, each point is represented as the mean standard deviation (± SD), n=4

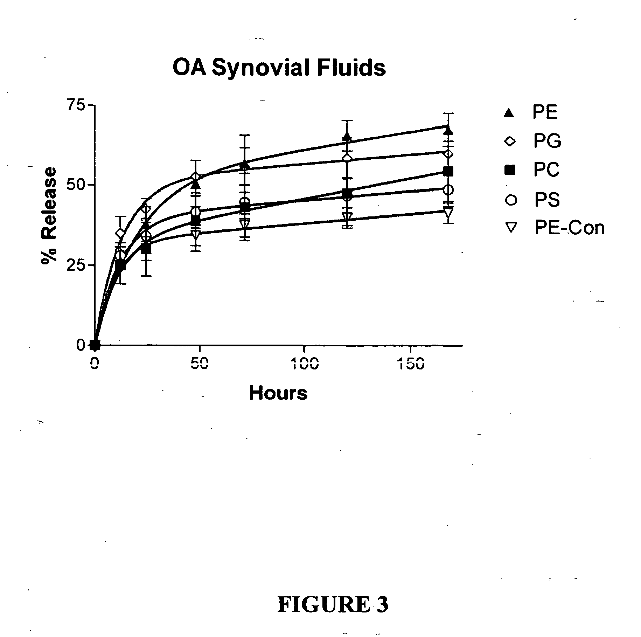

Figure 3 shows a kinetic profile for *in vitro* release of [³H]GlcNAc entrapped in different liposomal formulations into the synovial fluid from 3 OA patients. The amount of [³H]GlcNAc released from liposomes was measured in the supernatant and calculated as the percentage of the initially-entrapped compound released. In Fig. 3, the error bars represent a mean standard deviation (SD) for three data points.

Various liposomal formulations based on PC/Chol (1:1) or DSPC/Chol/DSPE (5:5:1) prolonged the GlcNAc retention at the rat knee joints after intra-articular administration.

### EXAMPLE 3

### IN VIVO RETENTION OF GlcNAc AFTER INTRA-ARTICULAR ADMINISTRATION

50 µl of GlcNAc solution (unlabeled 2.69 mg/ml, labeled 5 µCi/ml, unlabeled:labeled molecule 10,000:1) was injected into knee joint of six rats. These rats (2 animals/time point) were sacrificed at 0, 30 min, and 2 hr after administration of a GlcNAc solution. Two other two rats were injected with normal saline as negative controls. All joints were removed and homogenized with 5 ml of normal saline at 16,000 rpm for about 30 sec. 100 µl of the tissue homogenate was added to 500 µl tissue solubilizer. The solution was incubated at 50 °C for 16 hrs, mixed with 30 µl of glacial acetic acid and 5 ml of liquid scintillation cocktail. The [³H]GlcNAc activity was measured by a liquid scintillation counter.

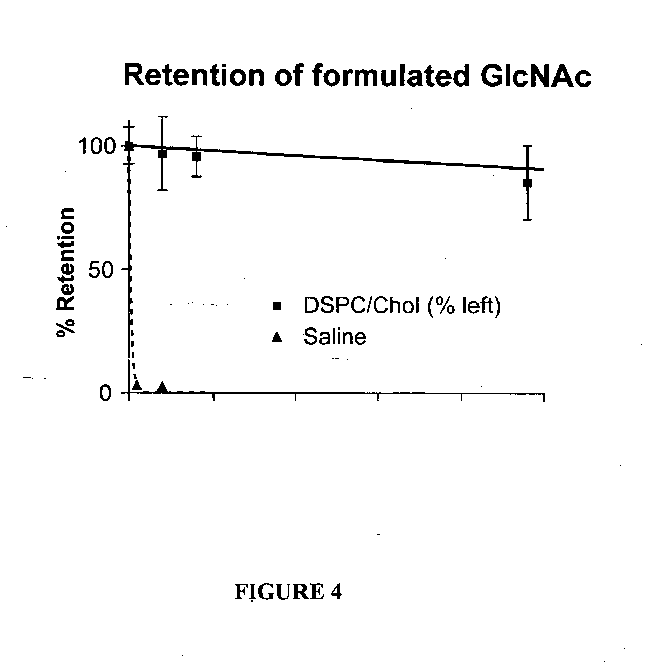

Fourteen rats were used for each liposomal formulation. 50 µl of liposome (prepared as disclosed herein, 1.13 µCi/ml, GlcNAc concentration was 0.61 mg/ml, unlabeled:labeled molecule 10,000:1) was injected into each knee joint in twelve rats. Another two rats were injected with 50 µl of normal saline as negative controls. Animals (2 rats/time point) were sacrificed at time points corresponding to 0, 1, 3, 7, 14, 21, and 28 days after administration of GlcNAc. The knee joints were removed and [³H]GlcNAc activity was determined as disclosed herein. The results from this study, depicted in Figure 4, demonstrated a profound prolongation of intra-articular half life.

Figure 4 shows a kinetic profile for the retention of GlcNAc in rat knee joints after a single intra-articular administration of GlcNAc entrapped in a liposomal formulation comprising DSPC/Chol = 5:5 (points and fitted line) or in a normal saline solution (dashed line). Each point is represented as the mean ± SD, n=4. The half-life of the GlcNAc saline solution in rat knee joints was very rapid (4.1 minutes) and the line runs very close to the abscissa in Figure 4). The half life of lipid formulated GlcNAc in rat joints was 173 hr. Without formulation, GlcNAc moved out of the rat joint rapidly after the intra-articularly administered GlcNAc.

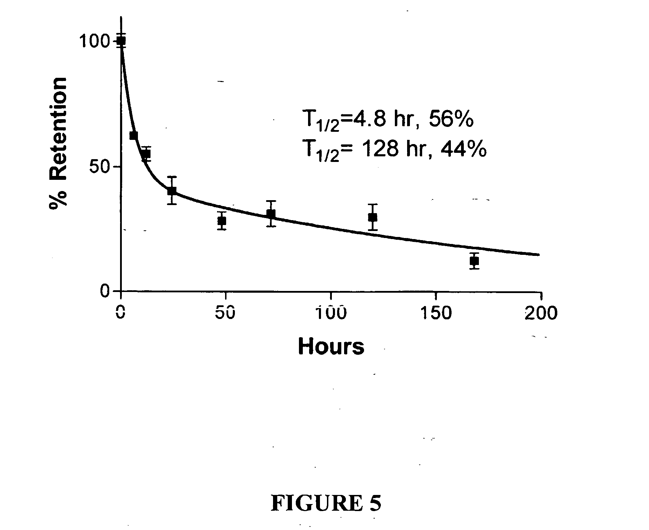

Figure 5 shows a kinetic profile for the retention of GlcNAc in rat knee joints after a single intra-articular administration of GlcNAc entrapped in liposomal formulation comprising DSPC/Chol DSPE = 5:5:1. Each point is represented as the mean ± SD, n = 4. A biphasic release curve was observed for this liposomal formulation corresponding to two distinct phases of release. The calculated half-lives of GlcNAc release from this liposomal formulation in rat joints were 4.8 hr and 128 hr, respectively.

### EXAMPLE 4

### ANALGESIC EFFECT OF GlcNAc AFTER INTRA-ARTICULAR ADMINISTRATION

The purpose of this study was to investigate the analgesic effects of GlcNAc with or without liposomal formulations, injected intra-articularly into the joint of Wistar rats. Pain was induced by intra-articular administration of bradykinin. Adult male Wistar rats with a weight range from 220g to 250g were obtained from Laboratory Animals Centre, National University of Singapore. Bradykinin and GlcNAc were obtained from Sigma. The liposomes were prepared as described above.

50 µl of GlcNAc solution or an GIcNAc formulated liposome suspension was injected into a rat joint cavity. An equal volume of saline was given intra-articularly as a control. 50 µl of bradykinin (60 µg/ml or at the indicated concentration) was given intra-articularly at different time points following the administration of GlcNAc.

Changes in walking behavior caused by intra-articular injection of bradykinin were used to define induced pain criteria in rats. The methods used to observe the pain response are defined in Table 2. The degree of pain was assigned a score according to the criteria in Table 3.

**Table 2**

| PAIN CRITERIA | | |
|---|---|---|
| Walking behavior | Grade | Indication |
| Normal | normal | No changes in walking |
| Lameness | Lam-I | Lameness alone for 20 seconds or less |
| | Lam-II | Lameness alone for more than 20 sec |
| Pulling | Pul-I | Only transient pulling up of the |
| | | injected leg (five sec or less) |
| | Pul-II | Pulling up of the injected leg, followed |
| | | by lameness |
| Three legs | Thr-I | Transient (for five seconds or less) |
| | | walking on three legs, followed by |
| | | lameness |
| | Thr-II | Walking on three legs for more than |
| | | five sec, followed by lameness |

**Table 3**

| BEHAVIOR GRADING CRITERIA FOR EVALUATING PAIN REACTION | | |
|---|---|---|
| Grade of Pain | Walking behavior | Evaluation score |
| None | Normal walking | 0 |
| Slight | Lam-I | 1 |
| Mild | Lam-II or Pul-I | 2 |
| Moderate | Pul-II or Thr-1 | 3 |
| Severe | Thr-II | 4 |

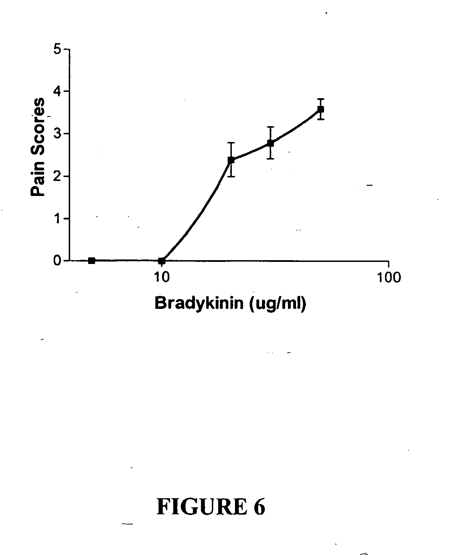

Figure 6 shows a dose response of bradykinin in rats after intra-articular administration as described herein. Higher pain scores correlate with the amount of injected Bradykinin.

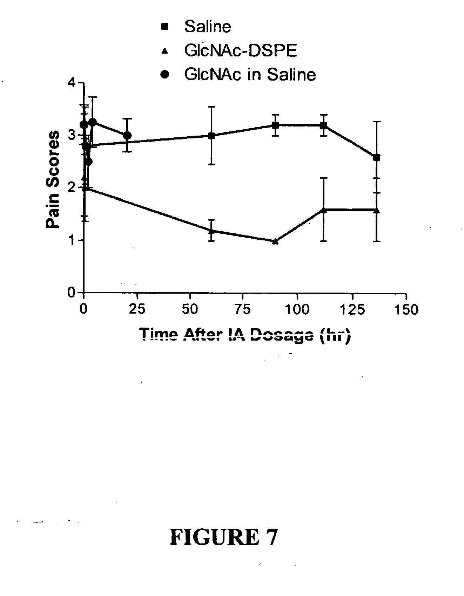

Figure 7 shows analgesic effects of GlcNAc either in saline or entrapped in a liposomal formulation. GlcNAc was liposomally formulated with a lipid composition of DSPC/Chol/DSPE (5:5:1). Liposomal formulation of GlcNAc showed lower pain scores demonstrating analgesic effects on bradykinin-induced pain in Wistar rats. GlcNAc itself without formulation showed small, but statistically insignificantly, analgesic effects.

It should be noted that steps recited in any method claims below do not necessarily need to be performed in the order that they are recited. Those of ordinary skill in the art will recognize variations in performing the steps from the order in which they are recited. For example, in certain embodiments, steps may be performed simultaneously. The accompanying claims should be constructed with these principles in mind.

## Claims

1. An injectable formulation comprising an entrapped active ingredient comprising an aminosugar as active ingredient for intra-articular treatment of a disease or condition in animals or humans.

2. The formulation according to claim 1 wherein said active ingredient is an aminosugar selected from N-acetylglucosamine, glucosamine, galactosamine, Nacetylgalactosamine or iminocyclitol and pharmaceutically acceptable salts thereof.

3. The formulation according to claim 1 wherein said active ingredient is entrapped by liposomes.

4. The formulation according to claim 1 wherein said active ingredient is entrapped by microspheres.

5. The formulation according to claim 1 wherein said active ingredient is entrapped by solid-lipid nanoparticles.

6. The formulation according to claim 1 further comprising one or more polymeric carriers selected from the group consisting of hyaluronic acid, polyethylene glycol, copolymers of polyethylene glycol and poly (lactic/glycolic acid.), copolymers of poly (ethylene glycol-γ- (DL-lactic acid-co-glycolic acid), alginate gels, chitins, and chitosans.

7. The formulation of claim 6, wherein said hyaluronic acid has an average molecular weight of about 400 to about 10,000,00 Daltons.

8. The formulation according to claim1, wherein the formulation is analgesic.

9. An injectable formulation comprising an aminosugar entrapped by liposomes, wherein said liposomes comprise one or more lipids selected from distearoyl L-α-phosphatidylcholine, distearoylL-α-phosphatidyl-ethanolamine, distearoyl L--α-phosphatidyl-ethanolamine conjugated with methoxypolyethylene glycol, distearoyl L-α-phosphatidyl-DL-glycerol, and dipalmitoyl L-α-phosphatidyl-L-serine.

10. The formulation according to claim 9 wherein said aminosugar is selected from N-acetylglucosamine, glucosamine, galactosamine, N-acetylgalactosamine or iminocyclitol and pharmaceutically acceptable salts thereof.

11. The formulation according to claim 9 wherein said liposomes are unilamellar or multilamellar and have an average diameter of 100 nm or greater.

12. An injectable formulation for intra-articular treatment of arthritis comprising an aminosugar entrapped by microspheres, wherein said microspheres comprise poly (lactic acid-glycolic acid) copolymers and pharmaceutically acceptable salts thereof.

13. Use of a therapeutically effective amount of an entrapped aminosugar as a controlled release formulation for the preparation of a medicament for treating arthritis, cartilage-related sports injury, or cartilage degradation; wherein said controlled release formulation comprises at least one of liposomes, microspheres, or solid-lipid nanoparticles.

14. The use according to claim 13 further comprising alleviating pain.

15. The use according to claim 13, wherein said formulation is topically administered in the form of a pharmaceutically acceptable gel, cream, lotion, or patch.

16. The use according to claim 13 wherein said arthritis is osteoarthritis or rheumatoid arthritis.

17. Use of a therapeutically effective amount of an aminosugar as a liposomal formulation for the preparation of a medicament for treating arthritis in a mammal.

18. The use of claim 17, wherein the liposomal formulation comprises one or more lipids selected from the group consisting of distearoyl L-α-phosphatidylcholine, distearoyl L-α-phosphatidyl-ethanolamine, distearoyl L-α-phosphatidylethanolamine conjugated with methoxypolyethylene glycol, distearoyl L-α-phosphatidyl-DL-glycerol, and dipalmitoyl L-α-phosphatidyl-L-serine.

19. The use according to claim 17, wherein said liposomal formulation provides pain relief.

20. Use of a therapeutically effective amount of N-acetylglucosamine as a liposomal formulation, wherein said liposomes comprise distearoyl L-α-phosphatidylcholine, distearoyl L-α-phosphatidylethanolamine, distearoylL-α-phosphatidyl-ethanolamine conjugated with methoxypolyethylene glycol, distearoyl L-α-phosphatidyl-DL-glycerol, or dipalmitoyl L-α-phosphatidyl-L-serine or any combination thereof for the preparation of a medicament for treating arthritis in a mammal by intra-articular injection.

## Patentansprüche

1. Injizierbare Zubereitung, umfassend eine eingeschlossenen Wirkstoff, der einen Aminozucker als Wirkstoff zur intraartikulären Behandlung einer Krankheit oder eines Zustandes bei Tieren oder Menschen umfasst.

2. Zubereitung nach Anspruch 1, wobei der Wirkstoff ein Aminozucker ist, ausgewählt aus N-Acetylglucosamin, Glucosamin, Galactosamin, N-Acetylgalctosamin oder Iminocyclitol und pharmazeutisch verträglichen Salzen davon.

3. Zubereitung nach Anspruch 1, wobei der Wirkstoff in Liposomen eingeschlossen ist.

4. Zubereitung nach Anspruch 1, wobei der Wirkstoff in Mikrosphären eingeschlossen ist.

5. Zubereitung nach Anspruch 1, wobei der Wirkstoff in festen Lipidnanopartikeln *(solid-lipid nanoparticles)* eingeschlossen ist.

6. Zubereitung nach Anspruch 1, weiterhin umfassend einen oder mehrere polymere Träger ausgewählt aus der Gruppe bestehend aus Hyaluronsäure, Polyethylenglykol, Copolymeren aus Polyethylengylkol und Poly-(Milch/GlycolSäure), Copolymeren aus Poly-(Ethylenglycol-y-(DL-Milchsäure-Coglycolsäure), Alginatgelen, Chitinen und Chitosanen.

7. Zubereitung nach Anspruch 6, wobei die Hyaluronsäure eine mittlere Molekülmasse von ungefähr 400 bis ungefähr 10000,00 Dalton hat.

8. Zubereitung nach Anspruch 1, wobei die Zubereitung analgetisch ist.

9. Injizierbare Zubereitung, umfassend einen in Liposomen eingeschlossenen Aminozucker, wobei die Liposomen ein oder mehrere Lipide umfassen, ausgewählt aus Distearoyl-L-α-phosphatidylcholin, Distearoyl-L-α-phosphatidylethanolamin, mit Methoxypolyethylenglcol konjugiertes Distearoyl-L-αphosphatidyl-ethanolamin, Distearoyl-L-α-phosphatidyl-DL-glycerin und Dipalmitoyl-L-α-phosphatidyl-L-serin.

10. Zubereitung nach Anspruch 9, wobei der Aminozucker ausgewählt ist aus N-Acetylglucosamin, Glucosamin, Galactosamin, N-Acetylgalactosamin oder Iminocyclitol und pharmazeutisch verträglichen Salzen davon.

11. Zubereitung nach Anspruch 9, wobei die Liposomen unilamellar oder mulitlamellar sind und einen mittleren Durchmesser von 100 nm oder mehr haben.

12. Injizierbare Zubereitung zur intraartikulären Behandlung von Arthritis, umfassend einen in Mikrosphären eingeschlossenen Aminozucker, wobei die Mikrosphären Poly-(Milchsäure-Glycolsäure)-Coplymere und pharmazeutisch verträgliche Salze davon umfassen.

13. Verwendung einer therapeutisch wirksamen Menge eines eingeschlossenen Aminozuckers als eine kontrolliert freigesetzte Zubereitung zur Herstellung eines Medikaments zur Behandlung von Arthritis, knorpelbezogenen Sportverletzungen, oder Knorpelabbau, wobei die kontrolliert freigesetzte Zubereitung wenigstens Liposomen, Mikrosphären oder feste Lipidnanopartikel umfasst.

14. Verwendung nach Anspruch 13, weiterhin umfassend Schmerzlinderung.

15. Verwendung nach Anspruch 13, wobei die Zubereitung topisch in Form eines pharmazeutisch verträgliches Gels, einer pharmazeutisch verträglichen Creme, einer pharmazeutisch verträglichen Lotion oder eines pharmazeutisch verträglichen Pflasters verabreicht wird.

16. Verwendung nach Anspruch 13, wobei die Arthritis Osteoarthritis oder rheumatoide Arthritis ist.

17. Verwendung einer therapeutisch wirksamen Menge eines Aminozuckers als liposomale Zubereitung zur Herstellung eines Medikaments zur Behandlung von Arthritis bei einem Säuger.

18. Verwendung nach Anspruch 17, wobei die liposomale Zubereitung ein oder mehrere Lipide umfasst, ausgewählt aus der Gruppe bestehend aus Distearoyl-L-α-phosphatidylcholin, Distearoyl-L-α-phosphatidyl-ethanolamin, mit Methoxypolyethylengylcol konjugiertes Distearoyl-L-α-phosphatidyl-ethanolamin, Distearoyl-L-α-phosphatidyl-DL-glycerin und Dipalmitoyl-L-α-phosphatidyl-L-serin.

19. Verwendung nach Anspruch 17, wobei die liposomale Zubereitung Schmerzen lindert.

20. Verwendung einer therapeutisch wirksamen Menge von N-Acetylglucosamin als liposomale Zubereitung, wobei die Liposomen Distearoyl-L-α-phosphatidylcholin, Distearoyl-L-α-phosphatidyl-ethanolamin, mit Methoxypolyethylenglcol konjugiertes Distearoyl-L-α-phosphatidyl-ethanolamin, Distearoyl-L-αphosphatidyl-DL-glycerin oder Dipalmitoyl-L-α-phosphatidyl-L-serin oder jede beliebige Kombination umfassen, zur Herstellung eines Medikamentes zur Behandlung von Arthritis bei einem Säuger durch intraartikuläre Injektion.

## Revendications

1. Formule injectable comprenant un ingrédient actif piégé comprenant un sucre aminé en tant qu'ingrédient actif pour le traitement intra-articulaire d'une maladie ou d'un état chez des animaux ou des humains.

2. Formule selon la revendication 1, dans laquelle ledit ingrédient actif est un sucre aminé choisi parmi la N-acétylglucosamine, la glucosamine, la galactosamine, la N-acétylgalactosamine ou l'iminocyclitol et leurs sels pharmaceutiquement acceptables.

3. Formule selon la revendication 1, dans laquelle ledit ingrédient actif est piégé par des liposomes.

4. Formule selon la revendication 1, dans laquelle ledit ingrédient actif est piégé par des microsphères.

5. Formule selon la revendication 1, dans laquelle ledit ingrédient actif est piégé par des nanoparticules solides à base de lipides.

6. Formule selon la revendication 1, comprenant en outre un ou plusieurs supports polymères choisis dans le groupe constitué par l'acide hyaluronique, le polyéthylène glycol, les copolymères de polyéthylène glycol et de poly(acide lactique/acide glycolique), les copolymères de poly(éthylène glycol-γ-(acide DL-lactique-co-acide glycolique)), les gels d'alginate, les chitines et les chitosanes.

7. Formule selon la revendication 6, dans laquelle ledit acide hyaluronique possède un poids moléculaire moyen d'environ 400 à environ 10 000 00 Daltons.

8. Formule selon la revendication 1, dans laquelle la formule est analgésique.

9. Formule injectable comprenant un sucre aminé piégé par des liposomes, dans laquelle lesdits liposomes comprennent un ou plusieurs lipides choisis parmi la distéaroyl L-α-phosphatidylcholine, la distéaroyl L-α-phosphatidyl-éthanolamine, la distéaroyl L-α-phosphatidyl-éthanolamine conjuguée avec le méthoxypolyéthylène glycol, le distéaroyl L-α-phosphatidyl-DL-glycérol et la dipalmitoyl L-α-phosphatidyl-L-sérine.

10. Formule selon la revendication 9, dans laquelle ledit sucre aminé est choisi parmi la N-acétylglucosamine, la glucosamine, la galactosamine, la N-acétylgalactosamine ou l'iminocyclitol et leurs sels pharmaceutiquement acceptables.

11. Formule selon la revendication 9, dans laquelle lesdits liposomes sont unilamellaires ou plurilamellaires et possèdent un diamètre moyen de 100 nm ou plus.

12. Formule injectable pour le traitement intra-articulaire d'une arthrite comprenant un sucre aminé piégé par des microsphères, dans laquelle lesdites microsphères comprennent des copolymères de poly(acide lactique - acide glycolique) et leurs sels pharmaceutiquement acceptables.

13. Utilisation d'une quantité thérapeutiquement efficace d'un sucre aminé piégé sous la forme d'une formule à libération contrôlée pour la préparation d'un médicament destiné au traitement de l'arthrite, de blessures sportives associées au cartilage ou de la dégradation du cartilage ; dans laquelle ladite formule à libération contrôlée comprend au moins un élément parmi des liposomes, des microsphères ou des particules solides à base de lipides.

14. Utilisation selon la revendication 13, comprenant en outre le soulagement d'une douleur.

15. Utilisation selon la revendication 13, dans laquelle ladite formule est administrée par voie topique sous la forme d'un gel, d'une crème, d'une lotion ou d'un timbre transdermique pharmaceutiquement acceptable.

16. Utilisation selon la revendication 13, dans laquelle ladite arthrite est l'ostéoarthrite ou la polyarthrite rhumatoïde.

17. Utilisation d'une quantité thérapeutiquement efficace d'un sucre aminé sous la forme d'une formule de liposomes pour la préparation d'un médicament destiné au traitement de l'arthrite chez un mammifère.

18. Utilisation selon la revendication 17, dans laquelle la formule de liposomes comprend un ou plusieurs lipides choisis dans le groupe constitué par la distéaroyl L-α-phosphatidylcholine, la distéaroyl L-α-phosphatidyl-éthanolamine, la distéaroyl L-α-phosphatidyl-éthanolamine conjuguée avec le méthoxypolyéthylène glycol, le distéaroyl L-α-phosphatidyl-DL-glycérol et la dipalmitoyl L-α-phosphatidyl-L-sérine.

19. Utilisation selon la revendication 17, dans laquelle ladite formule de liposomes permet de soulager une douleur.

20. Utilisation d'une quantité thérapeutiquement efficace de N-acétylglucosamine sous la forme d'une formule de liposomes, dans laquelle lesdits liposomes comprennent la distéaroyl L-a-phosphatidylcholine, la distéaroyl L-α-phosphatidyl-éthanolamine, la distéaroyl L-α-phosphatidyl-éthanolamine conjuguée avec le méthoxypolyéthylène glycol, le distéaroyl L-α-phosphatidyl-DL-glycérol ou la dipalmitoyl L-α-phosphatidyl-L-sérine ou n'importe quelle combinaison de ceux-ci pour la préparation d'un médicament destiné au traitement de l'arthrite chez un mammifère par injection intra-articulaire.
